Europäisches Patentamt

European Patent Office

Office européen des brevets

(19)

(11) Numéro de publication : **0 346 189 B1**

(12) **FASCICULE DE BREVET EUROPEEN**

(45) Date de publication du fascicule du brevet :
**19.08.92 Bulletin 92/34**

(51) Int. Cl.$^5$ : **A61K 7/48**

(21) Numéro de dépôt : **89401506.4**

(22) Date de dépôt : **01.06.89**

(54) **Compositions dermatocosmétologiques pour le traitement de la sénescence cutanée et la prévention des rides.**

Le dossier contient des informations techniques présentées postérieurement au dépôt de la demande et ne figurant pas dans leprésent fascicule.

(30) Priorité : **03.06.88 FR 8807425**

(43) Date de publication de la demande :
**13.12.89 Bulletin 89/50**

(45) Mention de la délivrance du brevet :
**19.08.92 Bulletin 92/34**

(84) Etats contractants désignés :
**AT BE CH DE ES GB GR IT LI LU NL SE**

(56) Documents cités :
**WO-A-88/06034**
**FR-A- 1 355 783**
**FR-A- 1 361 925**
**FR-A- 1 489 740**
**FR-A- 2 511 243**
**FR-A- 2 590 169**
**FR-A- 2 609 393**

(56) Documents cités :
**HUGO JANISTYN: "Handbuch der Kosmetika und Riechstoffe", édition 2, vol. 3: "Die Körperpflegemittel", 1973, pages 620-631,Dr. Alfred Hüthig Verlag, Heidelberg, DE PATENT ABSTRACTS OF JAPAN, vol. 8, no. 255 (C-253)[1692], 21 novembre 1984**

(73) Titulaire : **PIERRE FABRE COSMETIQUE**
**45, Place Abel Gance**
**F-92100 Boulogne (FR)**

(72) Inventeur : **Cousse, Henri**
**La Foun de los Novios Chemin de Lastinos**
**F-81100 Castres (FR)**
Inventeur : **Marty, Jean-Claude**
**104, chemin des Fourches**
**F-81100 Castres (FR)**
Inventeur : **Mouzin, Gilbert**
**11, rue des Pénitents blancs**
**F-31000 Toulouse (FR)**
Inventeur : **Navarro, Roger**
**Riveneuve du Crieu Route de Belpech**
**F-09100 Pamiers (FR)**

(74) Mandataire : **Ahner, Francis et al**
**CABINET REGIMBEAU, 26, avenue Kléber**
**F-75116 Paris (FR)**

EP 0 346 189 B1

EP 0 346 189 B1

## Description

La présente invention, réalisée au Centre de Recherche Dermatologique et Cosmétologique PIERRE FABRE, concerne de nouvelles formulations topiques utilisables en dermatologie et cosmétologie. Ces formulations sont à base d'une association de principes actifs, qui potentialisent la régénération et la protection du collagène. Les principes actifs sont principalement constitués par des polynucléotides et de l'hydroxyproline, éventuellement en association avec la vitamine E et/ou ses dérivés. L'utilisation d'extraits de cartilages (qui apportent l'hydroxiproline) en compositions cosmétoliques pour empêcher l'apparition de formations fibreuses cicatricielles est décrit dans la demande de brevet FR-A-1355783.

Dans l'état de la technique antérieure, les polynucléotides et plus particulièrement l'acide désoxyribonucléique sont largement utilisés en cosmétologie.

Les travaux les plus récents ont montré que l'utilisation par voie injectable de l'acide désoxyribonucléique ADN sous forme hautement polymérisée (ADN HP) donnait des résultats supérieurs à ceux obtenus avec le collagène dans le traitement des rides.

Les principes actifs utilisés dans les formulations topiques selon la présente invention permettent d'obtenir des résultats performants dans le traitement ou la prévention du vieillissement.

Conformément à la présente invention, la composition dermatocosmétologique contient une association d'un polynucléotide et de 4 hydroxyproline.

Le polynucléotide est en particulier constitué par de l'acide désoxyribonucléique (ADN), et de préférence de l'ADN hautement polymérisé (ADN HP), c'est-à-dire présentant une masse moléculaire comprise entre 500 000 et 1 500 000, de préférence entre 800 000 et 1 200 000.

La composition selon l'invention contient avantageusement de 0,5 à 5% en poids d'acide désoxyribonucléique.

La 4 hydroxyproline est de préférence utilisée sous la forme naturelle, c'est-à-dire sous la forme de son isomère L. Cette hydroxyproline peut notamment être apportée à la composition de l'invention, sous la forme d'un extrait de cartilage.

La composition de l'invention contient de façon avantageuse de 1 à 10 % en poids d'hydroxyproline par exemple apportés par un extrait de cartilage ajouté à la composition à hauteur de 3 à 30% en poids.

Selon une autre caractéristique importante de la présente invention, le rapport pondéral entre le polynucléotide et la 4 hydroxyproline est sensiblement compris entre 1/2 et 1/4.

Selon une caractéristique additionnelle de la présente invention, la composition contient en outre de la vitamine E ou l'un de ses dérivés, en particulier la vitamine E sous la forme acétate.

Les formulations topiques de la nouvelle association de principes actifs selon la présente invention sont illustrées par les exemples suivants :

**Exemple 1** : Emulsion fluide H/E de type ionique contenant un émulsionnement phosphaté, une phase grasse réduite.

| Principes actifs : | | |
|---|---|---|
| | ADN HP | 0,5 g |
| | 4 hydroxyproline | 1 g |
| | Vitamine E acétate | 1 g |
| | qsp | 100 g |

**Exemple 2** : Emulsion crème H/E

| Principes actifs : | | |
|---|---|---|
| | ADN HP | 1 g |
| | 4 hydroxyproline | 4 g |
| | Vitamine E acétate | 5 g |
| | qsp | 100 g |

2

**Exemple 3** : Emulsion fluide (exemple comparatif)

| Principes actifs : | ADN | 0,5 g |
|---|---|---|
| | Vitamine E acétate | 1 g |
| | qsp | 100 g |

**Exemple 4** : EMulsion crème (exemple comparatif)

| Principes actifs : | ADN HP | 1 g |
|---|---|---|
| | Vitamine E acétate | 5 g |
| | qsp | 100 g |

**Exemple 5** : gel

| Principes actifs : | Extrait de cartilage titré à 30% en 4 hydroxyproline | 3 g |
|---|---|---|
| | ADN | 0,5 g |
| | qsp | 100 g |

## ETUDE TOXICOLOGIQUE

Les essais ont été effectués de façon systématique sur les cinq formulations précédentes.

a) tolérance cutanée

Elle a été recherchée, pour chacune des formulations, sur 5 cobayes albinos adultes. L'application de produit 3 fois par semaine pendant 2 semaines, sur le flanc préalablement tondu, ne provoque aucune réaction cutanée appréciable.

b) tolérance oculaire

Elle a été réalisée sur des lapins albinos, après instillation dans le sac conjonctival de 2 gouttes de solution (concentration de 1 à 5%). On observe le comportement de l'animal pendant 3 minutes ; pour tous les animaux traités aucune différence n'a été constatée comparativement à un lot d'animaux témoin.

## PHARMACOLOGIE

Culture cellulaire

Les UV perturbent le métabolisme cellulaire, en particulier celui des fibroblastes. Ainsi, des fibroblastes provenant d'une zone cutanée quelconque et exposés aux UV perdent leur capacité de synthèse des macromolécules et plus particulièrement du collagène.

Selon les travaux de OIKARINEN A. et coll.: Connective tissue alterations in skin exposed to natural and therapeutic UV radiations; Photodermatology 2, 15-26 (1985).

On a observé dans les conditions expérimentales une forte potentialisation de la régénération du collagène due à la présence d'ADN et de 4 hydroxyproline, ainsi qu'une protection accrue de la dégradation en présence de vitamine E. Les résultats les plus intéressants ont été constatés avec les formulations des exemples 1 et 2.

CLINIQUE

Les formulations des exemples 1 à 3 ont été testées en clinique en utilisant une méthode d'appréciation de l'activité antirides.

La méthode d'analyse utilisée est la technique des empreintes cutanées associée à l'analyse macrophotographique.

Les résultats de cette étude effectuée sur 20 patients pendant 1 mois de traitement sont mentionnés dans le tableau suivant.

| Formulations | $t_o$ | $t_o + 10j$ | $t_o + 20j$ | $t_o + 30j$ |
|---|---|---|---|---|
| Exemple 1 | + | + + | + + | + + + |
| Exemple 2 | + | + + | + + + | + + + + |
| Exemple 3 | + | + | + | + |

+ : pas de modification des rides

++ : légère atténuation des rides

+++ : atténuation des rides

++++ : très forte atténuation des rides

Ces résultats confirment la potentialisation très nette de l'activité antirides de l'ADN HP en présence de 4 hydroxyproline.

**Revendications**

**Revendications pour les Etats contractants suivants: AT, BE, CH, DE, GB, IT, LI, LU, NL, SE**

1. Composition dermatocosmétologique pour le traitement du vieillissement cutané et la prévention des rides, caractérisée en ce qu'elle contient une association d'un polynucléotide et de 4 hydroxyproline dans un rapport pondéral sensiblement compris entre 1/2 et 1/4.

2. Composition selon la revendication 1, caractérisée en ce que le polynucléotide est de l'acide désoxyribonucléique (ADN).

3. Composition selon la revendication 2, caractérisée en ce que l'acide désoxyribonucléique est un ADN hautement polymérisé.

4. Composition selon l'une des revendications 1 à 3, caractérisée en ce qu'elle contient la vitamine E ou l'un de ses dérivés.

5. Composition selon la revendication 4, caractérisée en ce que le dérivé de la vitamine E utilisé est la vitamine E acétate.

6. Composition selon l'une des revendications 1 à 5, caractérisée en ce qu'elle contient de 0,5 à 5% d'acide désoxyribonucléique.

7. Composition selon l'une des revendications 1 à 6, caractérisée en ce qu'elle contient de 1 à 10% de 4 hydroxyproline.

8. Composition selon l'une des revendications 1 à 6, caractérisée en ce qu'elle contient de 3 à 30% d'extrait de cartilage.

**Revendications pour les Etats contractants suivants: ES, GR**

1. Procédé de préparation d'une composition dermatocosmétologique pour le traitement du vieillissement cutané et la prévention des rides, caractérisée en ce qu'elle implique le mélange d'un polynucléotide et de 4 hydroxyproline dans un rapport pondéral sensiblement compris entre 1/2 et 1 /4.

2. Procédé de préparation d'une composition selon la revendication 1, caractérisée en ce que le polynucléotide est de l'acide désoxyribonucléique (ADN).

3. Procédé de préparation d'une composition selon la revendication 2, caractérisée en ce que l'acide désoxyribonucléique est un ADN hautement polymérisé.

4. Procédé de préparation d'une composition selon l'une des revendications 1 à 3, caractérisée en ce que l'on ajoute de la vitamine E ou l'un de ses dérivés.

5. Procédé de préparation d'une composition selon la revendication 4, caractérisée en ce que le dérivé de la vitamine E utilisé est la vitamine E acétate.

6. Procédé de préparation d'une composition selon l'une des revendications 1 à 5, caractérisée en ce que l'on ajoute de 0,5 à 5% d'acide désoxyribonucléique par rapport au poids total de la composition.

7. Procédé de préparation d'une composition selon l'une des revendications 1 à 6, caractérisée en ce que l'on ajoute de 1 à 10% de 4 hydroxyproline par rapport au poids total de la composition.

8. Procédé de préparation d'une composition selon l'une des revendications 1 à 7, caractérisée en ce que l'on ajoute de 3 à 30% d'extrait de cartilage par rapport au poids total de la composition.

**Patentansprüche**

**Patentansprüche für folgende Vertragsstaaten: AT, BE, CH, DE, GB, IT, LI, LU, NL, SE**

1. Hautkosmetische Zusammensetzung für die Behandlung der Alterung (Erschlaffung) der Haut und die Verhinderung der Faltenbildung, dadurch gekennzeichnet, daß sie eine Assoziation aus einem Polynucleotid und 4-Hydroxyprolin in einem Gewichtsverhältnis von im wesentlichen zwischen 1/2 und 1/4 enthält.

2. Zusammensetzung nach Anspruch 1, dadurch gekennzeichnet, daß das Polynucleotid die Desoxyribonucleinsäure (DNA) ist.

3. Zusammensetzung nach Anspruch 2, dadurch gekennzeichnet, daß die Desoxyribonucleinsäure eine hoch polymerisierte DNA ist.

4. Zusammensetzung nach einem der Ansprüche 1 bis 3, dadurch gekennzeichnet, daß sie das Vitamin E oder eines seiner Derivate enthält.

5. Zusammensetzung nach Anspruch 4, dadurch gekennzeichnet, daß das verwendete Vitamin E-Derivat das Vitamin E-Acetat ist.

6. Zusammensetzung nach einem der Ansprüche 1 bis 5, dadurch gekennzeichnet, daß sie 0,5 bis 5 % Desoxyribonucleinsäure enthält.

7. Zusammensetzung nach einem der Ansprüche 1 bis 6, dadurch gekennzeichnet., daß sie 1 bis 10 % 4-Hydroxyprolin enthält.

8. Zusammensetzung nach einem der Ansprüche 1 bis 6, dadurch gekennzeichnet, daß sie 3 bis 30 % Knor-pelextrakt enthält.

**Patentansprüche für folgende Vertragsstaaten: ES, GR**

1. Verfahren zur Herstellung einer hautkosmetischen Zusammensetzung für die Behandlung der Alterung (Erschlaffung) der Haut und die Verhinderung der Faltenbildung, dadurch gekennzeichnet, daß es umfaßt das Mischen eines Polynucleotids mit 4-Hydroxyprolin in einem Gewichtsverhältnis von im wesentlichen zwischen 1/2 und 1/4.

2. Verfahren zur Herstellung einer Zusammensetzung nach Anspruch 1, dadurch gekennzeichnet, daß das Polynucleotid die Desoxyribonucleinsäure (DNA) ist.

3. Verfahren zur Herstellung einer Zusammensetzung nach Anspruch 2, dadurch gekennzeichnet, daß die Desoxyribonucleinsäure eine hoch polymerisierte DNA ist.

4. Verfahren zur Herstellung einer Zusammensetzung nach einem der Ansprüche 1 bis 3, dadurch gekennzeichnet, daß man das Vitamin E oder eines seiner Derivate zugibt.

5. Verfahren zur Herstellung einer Zusammensetzung nach Anspruch 4, dadurch gekennzeichnet, daß das verwendete Vitamin E-Derivat das Vitamin E-Acetat ist.

6. Verfahren zur Herstellung einer Zusammensetzung nach einem der Ansprüche 1 bis 5, dadurch gekennzeichnet, daß man 0,5 bis 5 % Desoxyribonucleinsäure, bezogen auf das Gesamtgewicht der Zusammensetzung, zugibt.

7. Verfahren zur Herstellung einer Zusammensetzung nach einem der Ansprüche 1 bis 6, dadurch gekennzeichnet, daß man 1 bis 10 % 4-Hydroxyprolin, bezogen auf das Gesamtgewicht der Zusammensetzung, zugibt.

8. Verfahren zur Herstellung einer Zusammensetzung nach einem der Ansprüche 1 bis 7, dadurch gekennzeichnet, daß man 3 bis 30 % Knorpelextrakt, bezogen auf das Gesamtgewicht der Zusammensetzung, zugibt.

## Claims

### Claims for the following Contracting States: AT, BE, CH, DE, GB, IT, LI, LU, NL, SE

1. A cosmetic skin composition for treatment of ageing skin and prevention of wrinkles, characterised in that it contains an association of a polynucleotide with 4 hydroxyproline in a ratio substantially between 1/2 and 1/4 by weight.

2. A composition according to claim 1, characterised in that the polynucleotide is deoxyribonucleic acid (DNA).

3. A composition according to claim 2, characterised in that the deoxyribonucleic acid is a highly-polymerised DNA.

4. A composition according to any of claims 1 to 3, characterised in that it contains vitamin E or one of its derivatives.

5. A composition according to claim 4, characterised in that the vitamin E derivative is vitamin E acetate.

6. A composition according to any of claims 1 to 5, characterised in that it contains 0.5 to 5% of deoxyribonucleic acid.

7. A composition according to any of claims 1 to 6, characterised in that it contains 1 to 10% of 4 hydroxyproline.

8. A composition according to any of claims 1 to 6, characterised in that it contains 3 to 30% of cartilage extract.

### Claims for the following Contracting States: ES, GR

1. A method of preparing a cosmetic skin composition for treatment of ageing skin and prevention of wrinkles, characterised in that it involves mixing a polynucleotide with 4 hydroxyproline in a ratio substantially between 1/2 and 1/4 by weight.

2. A method of preparing a composition according to claim 1, characterised in that the polynucleotide is deoxyribonucleic acid (DNA).

3. A method of preparing a composition according to claim 2, characterised in that the deoxyribonucleic acid is a highly-polymerised DNA.

4. A method of preparing a composition according to any of claims 1 to 3, characterised in that it contains vitamin E or one of its derivatives.

5. A method of preparing a composition according to claim 4, characterised in that the vitamin E derivative is vitamin E acetate.

6. A method of preparing a composition according to any of claims 1 to 5, characterised in that 0.5 to 5% of deoxyribonucleic acid is added relative to the total weight of the composition.

7. A method of preparing a composition according to any of claims 1 to 6, characterised in that 1 to 10% of 4 hydroxyproline is added relative to the total weight of the composition.

8. A method of preparing a composition according to any of claims 1 to 7, characterised in that 3 to 30% of cartilage extract is added relative to the total weight of the composition.